# EUROPEAN PATENT SPECIFICATION

(11) **EP 4 413 960 B1**
(45) Date of publication and mention of the grant of the patent: **11.06.2025**
(21) Application number: 24177369.6
(22) Date of filing: 26.01.2021
(51) Int. Cl.: A61F 5/453, A61F 5/455

(54) **URINE COLLECTION DEVICES ADJUSTABLE BETWEEN COMPACT AND EXTENDED CONFIGURATIONS, AND RELATED METHODS**
ZWISCHEN KOMPAKTEN UND ERWEITERTEN KONFIGURATIONEN VERSTELLBARE URINSAMMELVORRICHTUNGEN UND ZUGEHÖRIGE VERFAHREN
DISPOSITIFS DE COLLECTE D'URINE RÉGLABLES ENTRE DES CONFIGURATIONS COMPACTES ET ÉTENDUES, ET PROCÉDÉS ASSOCIÉS

(30) Priority: 29.01.2020 US 202062967158 P
(43) Date of publication of application: 14.08.2024
(62) Divisional of application: 21707459.0
(73) Proprietor: Purewick Corporation, El Cajon, California 92020 (US)
(72) Inventor: HUGHETT, SR., James David, Conyers, Georgia (US); DAW, Kyle, Smyrna, Georgia 30080 (US); HINESLEY, Hannah, Monticello, Georgia 31064 (US); FERNANDEZ, Rodrigo, Loganville, Georgia 30052 (US); HIETT, Ginger, Covington, Georgia 30016 (US); BOWLES, Caitlin, Atlanta, Georgia 30313 (US); MADIGAN, Henri, Monroe, Georgia 30656 (US); CISNEROS, Juan Alejandro Saavedra, Loganville, Georgia 30052 (US); SALIFU, Hassana, Roswell, Georgia 30076 (US); MYERS, Benjamin, Atlanta, Georgia 30308 (US)
(74) Representative: Hoffmann Eitle

(56) References cited:
- CN-A- 1 332 620
- US-B2- 9 456 937

## Description

### BACKGROUND

An individual may have limited or impaired mobility such that typical urination processes are challenging or impossible. For example, the individual may have surgery or a disability that impairs mobility. In another example, the individual may have restricted travel conditions such as those experience by pilots, drivers, and workers in hazardous areas. Additionally, fluid collection from the individual may be needed for monitoring purposes or clinical testing.

Bed pans and urinary catheters, such as a Foley catheter, may be used to address some of these circumstances. However, bed pans and urinary catheters have several problems associated therewith. For example, bed pans may be prone to discomfort, spills, and other hygiene issues. Urinary catheters be may be uncomfortable, painful, and may cause urinary tract infections. US 9 456 937 B2 discloses an urine collection device with a fluid impermeable barrier with a support member configured to retain the fluid impermeable barrier in an extended configuration and in a compact configuration so that the fluid impermeable barrier is adjustable between the extended configuration and the compact configuration.

Thus, users and manufacturers of urine collection devices continue to seek new and improved devices, systems, and methods to collect urine.

### SUMMARY

The invention is defined in claim 1 below. The dependent claims are directed to preferred embodiments and optional features of the invention. Disclosed herein are cylindrical urine collection devices. Such a urine collection device includes a fluid impermeable barrier and fluid permeable structure. The fluid impermeable barrier is adjustable between an extended configuration and a compact configuration. The fluid impermeable barrier also at least partially defines a chamber, an aperture configured to receive a conduit therethrough, and an opening. The fluid permeable structure is positioned within the chamber to extend across at least a portion of the opening. The fluid permeable structure is configured to wick fluid away from the opening.

As disclosed, a urine collection device includes a fluid impermeable barrier, a support member, and fluid permeable structure. The fluid impermeable barrier is adjustable between an extended configuration and a compact configuration. The fluid impermeable barrier at least partially defines a chamber, an aperture configured to receive a conduit therethrough, and an opening. The support member is secured to the fluid impermeable barrier. The support member is configured to retain the fluid impermeable barrier in the extended configuration and the compact configuration. The fluid permeable structure is positioned within the chamber to extend across at least a portion of the opening. The fluid permeable structure is configured to wick fluid away from the opening.

One disclosed urine collection device includes a fluid impermeable barrier which includes a distal end positioned distal to the aperture, and the fluid impermeable barrier tapers for at least about 2.5 cm towards the distal end. The fluid permeable structure is positioned within the chamber to extend across at least a portion of the opening, and the fluid permeable structure is configured to wick fluid away from the opening.

Features from any of the disclosed embodiments may be used in combination with one another, without limitation. In addition, other features and advantages of the present disclosure will become apparent to those of ordinary skill in the art through consideration of the following detailed description and the accompanying drawings.

### BRIEF DESCRIPTION OF THE DRAWINGS

In the drawings, identical reference numerals refer to identical or similar elements or features in different views or embodiments shown in the drawings.
**FIG. 1A** is a front plan view of a urine collection device in an extended configuration.
**FIG. 1B** is an isometric view of the urine collection device of **FIG. 1A** in a partially compact configuration.
**FIG. 1C** is a side view of the urine collection device of **FIG. 1A** in a compact configuration.
**FIG. 2A** is an isometric view of urine collection device.
**FIG. 2B** is a bottom plan view of the urine collection device of **FIG. 2A****.**
**FIG. 3** is a side view of a conduit used in a urine collection device.
**FIG. 4A** is an isometric view of a urine collection device according to the invention, in an extended configuration.
**FIG. 4B** is a front view of the urine collection device of **FIG. 4A** in a compact configuration.
**FIG. 5** is a block diagram of a system for collection urine.
**FIG. 6** is a flow diagram of a method for collection urine.

### DETAILED DESCRIPTION

The devices and systems disclosed herein are configured to collect fluids from an individual. The fluids collected by the urine collection devices may include at least one of urine, vaginal discharge, penile discharge, reproductive fluids, blood, sweat, or other bodily fluids. Urine collection devices described herein may be used in urine collection systems. The urine collection systems can include a urine collection device, a fluid storage container, and a portable vacuum source. Fluid *(e.g.,* urine or other bodily fluids) collected in the urine collection device may be removed from the urine collection device via a conduit which protrudes into an interior region of the urine collection device. For example, a first open end of the conduit may extend into the urine collection device to a reservoir therein. The second open end of the conduit may extend into the fluid storage container or the portable vacuum source. The suction force may be introduced into the interior region of the urine collection device via the first open end of the conduit responsive to a suction (e.g., vacuum) force applied at the second end of the conduit. The suction force may be applied to the second open end of the conduit by the portable vacuum source either directly or indirectly.

In some embodiments, the portable vacuum source may be disposed in or on the urine collection device. In such embodiments, the conduit may extend from the urine collection device and attach to the portable vacuum source at a first point therein. An additional conduit may attach to the portable vacuum source at a second point thereon and may extend out of the urine collection device, and may attach to the fluid storage container. Accordingly, a vacuum (e.g., suction) may be drawn through urine collection device via the fluid storage container. Fluid, such as urine, may be drained from the urine collection device using the portable vacuum source.

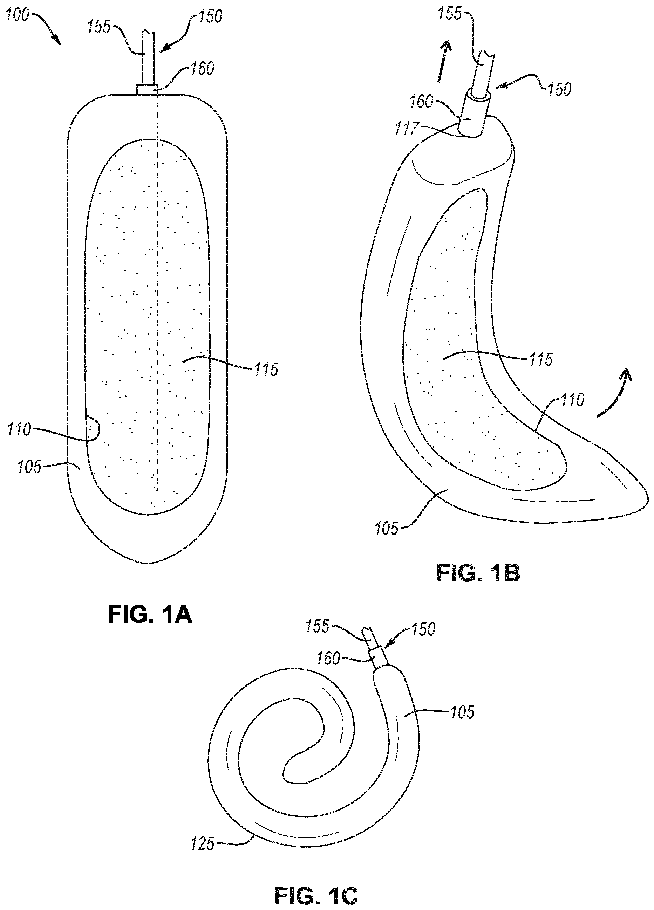

**FIG. 1A** is a front plan view of a urine collection device 100. The urine collection device 100 is a urine collection device 100 that is configured to receive fluid from a female or a male having a buried penis. The urine collection device 100 is generally smaller, and more compact that conventional urine collection devices, allowing the urine collection device 100 to be more discrete and used with a patient that is mobile or active. One or more components of the urine collection device 100 may be more flexible than conventional urine collection devices, thereby allowing one or more components of the urine collection device to be rolled into a small package. The device as illustrated does not offer the user a width selection facility as recited in claim 1 below.

The urine collection device 100 includes an elongated fluid impermeable barrier 105 that is adjustable between an extended configuration and a compact configuration. **FIG. 1A** shows the fluid impermeable barrier 105 in the extended configuration, **FIG. 1B** shows the fluid impermeable barrier 105 as the fluid impermeable barrier 105 is being manipulated to the compact configuration, and **FIG. 1C** shows the fluid impermeable barrier rolled up in the compact configuration. Although **FIG. 1C** shows the fluid impermeable barrier 105 rolled up into the compact configuration, in other designs, the fluid impermeable barrier 105 is configured to be folded up into the compact configuration, such that the fluid impermeable barrier 105 includes one or more folds in the compact configuration.

The fluid impermeable barrier 105 also defines a chamber (*e.g*., interior region), an opening 110, and an aperture 117 (shown in **FIG. 1B****)** that is sized and dimensioned to receive a conduit 150 therethrough. The fluid impermeable barrier 105 may temporarily store fluids that have been received through the opening 110 in the chamber. As such, the fluid impermeable barrier 105 substantially prevents the fluids from exiting the portions of the chamber that are spaced from the opening 110. The opening 110 defined by the fluid impermeable barrier 105 is formed in and extends through the fluid impermeable barrier 105, thereby enabling fluids to enter the chamber from outside of the urine collection device 100. The opening 110 can be configured to be positioned adjacent to a female urethra or positioned adjacent to the skin of a female over the urethra. With the urine collection device 100 positioned proximate to the female urethra or positioned adjacent to the skin of a female over the urethra, urine may enter the interior region or chamber of the urine collection device 100 via the opening 110. Accordingly, the urine collection device 100 is configured to receive the fluids into the chamber via the opening 110.

The fluid impermeable barrier 105 is flexible, allowing the urine collection device 100 to bend or curve when positioned against the body of a wearer in additional to the compact configuration (shown in **FIG. 1C****)** and the extended configuration (shown in **FIG. 1A****).** For example, the fluid impermeable barrier 105 may bend or curve against the body of an individual similar to the shape shown in **FIG. 1B****.** The fluid impermeable barrier 105 can be formed of any suitable fluid impermeable materials, such as a fluid impermeable polymer (*e.g*., silicone, polypropylene, polyethylene, polyethylene terephthalate, a polycarbonate, etc.), polyurethane films, thermoplastic elastomer, oil, another suitable material, or combinations thereof. In some designs, the fluid impermeable barrier includes a paper-like fluid impermeable material or a fluid impermeable fabric that is configured to be folded or rolled into a roll, as shown in **FIG. 1C****.** In some designs, the fluid impermeable barrier 105 includes a layer of material having a thickness that is less than about 1 mm, less than about 0.75 mm, less than about 0.5 mm, less than about 0.25 mm, about 0.1 mm to about 2 mm, about 0.1 mm to about 1.5 mm, about 0.1 mm to about 1 mm, about 0.1 mm to about 0.5 mm, about 0.1 mm to about 0.25 mm, about 0.25 mm to about 0.5 mm, about 0.5 mm to about 0.75 mm, about 0.75 mm to about 1 mm, about 1 mm to about 1.25 mm, or about 1.25 mm to about 1.5 mm. In some designs, the fluid impermeable barrier 105 includes a longitudinal length of less than about 20 cm, less than about 15 cm, less than about 10 cm, about 5 cm to about 10 cm, about 10 cm to about 15 cm, or about 15 cm to about 20 cm.

The fluid impermeable barrier 105 may include a substantially flat or planar area. That is, the fluid impermeable barrier 105 may include a substantially flat back side and front side defining the opening 110 having a substantially planar profile when the fluid impermeable barrier 105 is in the extended configuration shown in **FIG. 1A****.** The fluid impermeable barrier may include two rounded or planar sides extending between the back side and the front side. In other designs, the fluid impermeable barrier 105 may include a tubular shape (not shown).

The urine collection device also includes fluid permeable structure 115 positioned within the chamber defined by the fluid impermeable barrier 105 to extend across at least a portion of the opening 110. The fluid permeable structure 115 may be shaped generally complementary to the shape of the chamber of the fluid impermeable barrier 105. For example, when the fluid impermeable barrier 105 includes a generally flat or planar front side in the extended configuration, the fluid permeable structure 115 may include a generally flat or planar surface extending across at least a portion of the opening 110. When the fluid impermeable barrier 105 is generally tubular, the fluid permeable structure 115 may be generally tubular.

The fluid permeable structure 115 may be configured and sized to roll or fold with the fluid impermeable barrier 105 from the extended configuration to the compact configuration. For example, although not visible in the urine collection device 100 in **FIG. 1C****,** the fluid permeable structure 115 may be positioned within the chamber of the fluid impermeable barrier 105 when the fluid impermeable barrier 105 is in the rolled compact configuration shown in **FIG. 1C****.** In other designs, the fluid permeable structure may be positioned within the chamber of the fluid impermeable barrier 105 when the fluid impermeable barrier 105 is folded into the compact configuration. In still other designs, the fluid permeable structure 115 is removable from the chamber of the fluid impermeable barrier 105, and the fluid permeable structure 115 may be absent or separated from the fluid impermeable barrier 105 when the fluid impermeable barrier 105 is in the compact configuration.

The fluid permeable structure 115 can be configured to wick any fluid away from the opening 110, thereby preventing the fluid from escaping the chamber of the fluid impermeable barrier 105. The fluid permeable structure 115 also can wick the fluid generally towards an interior of the chamber. A portion of the fluid permeable structure 115 can define a portion of an outer surface of the urine collection device 100. Specifically, the portion of the fluid permeable structure 115 defining the portion of the outer surface of the urine collection device 100 can be the portion of the fluid permeable structure 115 exposed by the opening 110 defined by the fluid impermeable barrier 105 that contacts the user.

The fluid permeable structure 115 can include any material that can wick the fluid. The permeable properties referred to herein can be wicking, capillary action, diffusion, or other similar properties or processes, and are referred to herein as "permeable" and/or "wicking." Such "wicking" may exclude absorption into the wicking material. Put another way, substantially no absorption of fluid into the material may take place after the material is exposed to the fluid and removed from the fluid for a time. While no absorption is desired, the term "substantially no absorption" may allow for nominal amounts of absorption of fluid into the wicking material (e.g., absorbency), such as less than about 10 wt% of the dry weight of the wicking material, less than about 7 wt%, less than about 5 wt%, less than about 3 wt%, less than about 2 wt%, less than about 1 wt%, or less than about 0.5 wt% of the dry weight of the wicking material.

The fluid permeable structure 115 can include a one-way fluid movement fabric. As such, the fluid permeable structure 115 can remove fluid from the area around the buried penis or female urethra, thereby leaving the area and urethra dry. The fluid permeable structure 115 can enable the fluid to flow generally towards the conduit 150 within the chamber. The fluid permeable structure 115 can include a porous or fibrous material, such as hydrophilic polyolefin. In some designs, the fluid permeable structure 115 consists of or consists essentially of a porous or fibrous material, such as hydrophilic polyolefin. Examples of polyolefin that can be used in the fluid permeable structure 115 include, but are not limited to, polyethylene, polypropylene, polyisobutylene, ethylene propylene rubber, ethylene propylene diene monomer, or combinations thereof. Moreover, the fluid permeable structure 115 can be manufactured according to various manufacturing methods, such as molding, extrusion, or sintering. The fluid permeable structure 115 can include varying densities or dimensions.

In some designs, the fluid permeable structure 115 can include two or more layers of fluid permeable materials and include no more than two layers of material between the opening 110 and the conduit 150 positioned within the fluid permeable structure 115. For example, the urine collection device 100 can include a fluid permeable membrane covering or wrapped around a fluid permeable support of the fluid permeable structure 115, with both the fluid permeable membrane and the fluid permeable support being disposed in the chamber of the fluid impermeable barrier 105. The fluid permeable membrane can cover or extend across at least a portion *(e.g.,* all) of the opening 110. The fluid permeable membrane and the fluid permeable support can be configured to wick any fluid away from the opening 110, thereby preventing the fluid from escaping the chamber. The permeable properties referred to herein can be wicking, capillary action, diffusion, or other similar properties or processes, and are referred to herein as "permeable" and/or "wicking."

The fluid permeable membrane can also wick the fluid generally towards an interior of the chamber, as discussed in more detail below. The fluid permeable membrane can include any material that can wick the fluid. For example, the fluid permeable membrane can include fabric, such as a gauze *(e.g.,* a silk, linen, polymer based materials such as polyester, or cotton gauze), nylon (such as a spun nylon fibers), another soft fabric *(e.g.,* jersey knit fabric or the like), or another smooth fabric *(e.g.,* rayon, satin, or the like). Forming the fluid permeable membrane from gauze, soft fabric, and/or smooth fabric can reduce chaffing caused by the urine collection device 100. Other designs of fluid permeable membranes, fluid permeable supports, chambers, and their shapes and configurations are disclosed in U.S. Patent Application No. 15/612,325 filed on June 2, 2017; U.S. Patent Application No. 15/260,103 filed on September 8, 2016; U.S. Patent Application No. 15/611,587 filed on June 1, 2017; PCT Patent Application No. PCT/US19/29608, filed on April 29, 2019 In many realisations, the fluid permeable structure 115 includes an inner portion including a porous spun nylon fiber structure and an outer fluid permeable membrane including gauze.

The conduit 150 *(e.g.,* a tube) extends through the aperture 117 (shown in **FIG. 1B****)** into the chamber defined by the fluid impermeable barrier 105. For example, the conduit 150 may extend through the aperture 117 into the chamber. The conduit 150 provides fluid communication between the chamber and a fluid storage container (not shown) or a portable vacuum source (not shown). For example, the conduit 150 may directly or indirectly fluidly couple the chamber with the fluid storage container or the portable vacuum source. With the conduit 150 positioned within the fluid permeable structure 115, fluids received in the chamber can be removed through the conduit 150.

The conduit 150 may include a retractable conduit 150 configured to connect to an additional conduit providing fluid communication with a fluid storage container (not shown) or a portable vacuum source (not shown). The retractable conduit 150 includes a first portion 160 positioned at least partially within the chamber and a second portion 155 slidably connected to the first portion 160. The second portion 155 is at least partially positioned outside the chamber and at least partially retractable within the chamber. For example, substantially all of the second portion 155 may retract within the first portion 160 of the conduit, with a lip on at least one of the first portion 160 or the second portion 155 preventing the second portion 155 from sliding all the way into the first portion 160, or vice versa. The first portion 160 also may include a lip preventing the first portion 160 from sliding entirely into the chamber of the fluid impermeable barrier 105. The second portion 155 may slide within the first portion 160, or the first portion 160 may slide within the second portion 155.

In use, the conduit 150 may be retracted with the second portion 155 positioned at least partially within the chamber when the fluid impermeable barrier 105 is in the compact configuration. With the second portion 155 retracted, the urine collection device 100 may require a smaller packaging volume, thereby allowing for more discrete and convenient packaging and transport of the urine collection device 100 in the compact configuration. When the fluid impermeable barrier 105 is unrolled or otherwise manipulated to the extended configuration shown in **FIG. 1A****,** the second portion 155 may be at least partially withdrawn from the chamber, thereby extending the conduit 150 to a greater length. The second portion 155 may then be fluidly coupled directly or indirectly to the vacuum source or the fluid storage container.

In use, the opening 110 of the urine collection device 100 may be positioned proximate to a female urethra to collect discharged urine or other bodily fluids through the opening 110 and into the fluid permeable structure 115. The discharged urine or other bodily fluids may be wicked through the fluid permeable structure 115 to the conduit 150 for removal from the urine collection device 100 through the conduit 150.

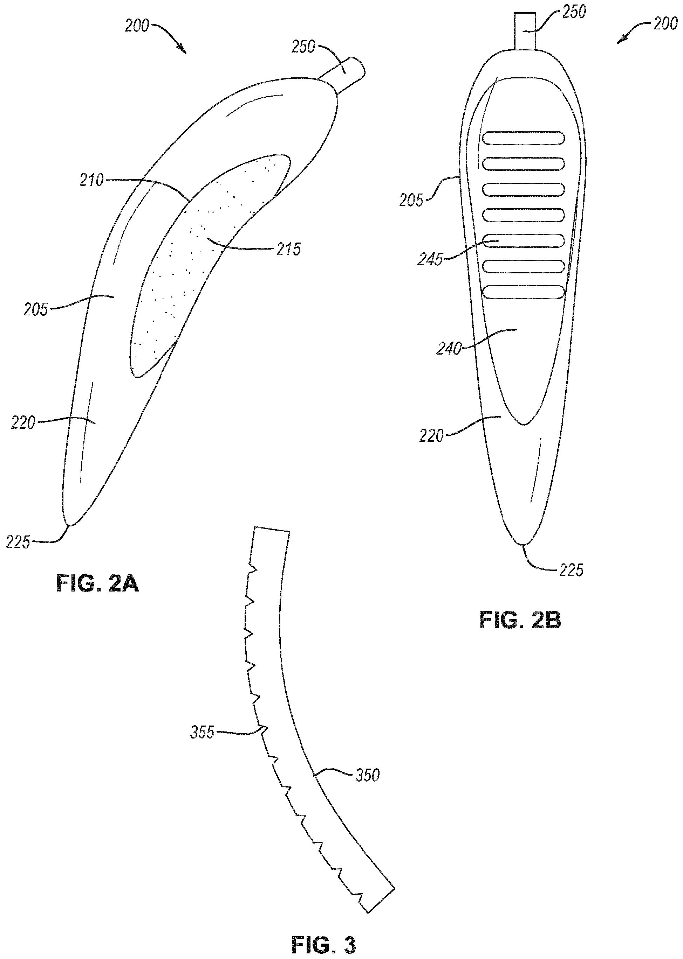

**FIG. 2A** is an isometric view of a urine collection device 200. The urine collection device 200 is an example of a urine collection device 200 that is configured to receive fluid from a female or a male having a buried penis. Unless otherwise noted, the urine collection device 200 can include any of the materials, components, and/or features described above in relation to the urine collection device 100. For example, the urine collection device 200 may be generally smaller, and more compact that conventional urine collection devices, allowing the urine collection device 200 to be more discreet and used with a patient that is mobile. One or more components of the urine collection device 200 may be more flexible than conventional urine collection devices, thereby allowing one or more components of the urine collection device to be rolled into a small package.

The urine collection device 200 includes an elongated fluid impermeable barrier 205. The fluid impermeable barrier 205 may include any of the materials described above in relation to the fluid impermeable barrier 105, thereby allowing the fluid impermeable barrier to be adjustable between any of the extended configurations and the compact configurations described herein. The fluid impermeable barrier 205 also defines a chamber (*e.g*., interior region), an opening 210, an aperture that is sized and dimensioned to receive a conduit 250 therethrough, and a distal end 225 that is distal to the aperture.

The fluid impermeable barrier 205 also may taper towards the distal end 225. In some designs, the fluid impermeable barrier may taper continuously over a portion of the fluid impermeable barrier 205. For example, a portion of the fluid impermeably barrier 205 may taper continuously from a distal end of the opening to the distal end 225 of the fluid impermeable barrier. In some designs, a portion of the fluid impermeable barrier 205 may continuously taper towards the distal end 225 for at least 1 inch (or about 2.5 cm), at least 1.5 inches (or about 3.8 cm), at least 2 inches (or about 5 cm), at least 2.5 inches (or about 6.4 cm), at least 3 inches (or about 7.6 cm), at least 3.5 inches (or about 8.9 cm), or at least 4 inches (or about 10.2 cm). In some designs, the fluid impermeable barrier 205 may be about 0.75 inch (about 1.9 cm) to about 1 inch (about 2.5 cm) wide before tapering to the distal end. Tapering to the distal end 225 provides a more comfortable fit for the individual using the urine collection device 200, especially if the individual is mobile. In the example shown in **FIG. 2A****,** the fluid impermeable barrier 205 is generally cylindrical or tubular, and the fluid impermeable barrier 205 is generally tubular or conical as the fluid impermeable barrier 205 tapers to the distal end 225. In some embodiments, the fluid impermeable barrier 205 may include one or more flat or planar areas that taper to the distal end 225.

The fluid impermeable barrier 205 may temporarily store fluids that have been received through the opening 210 in the chamber. As such, the fluid impermeable barrier 205 substantially prevents the fluids from exiting the portions of the chamber that are spaced from the opening 210. The opening 210 defined by the fluid impermeable barrier 205 is formed in and extends through the fluid impermeable barrier 205, thereby enabling fluids to enter the chamber from outside of the urine collection device 200. The opening 210 can be configured to be positioned adjacent to a female urethra or positioned adjacent to the skin of a female over the urethra, with the distal end 225 between the legs or gluteal cleft of the female. With the urine collection device 200 positioned proximate to the female urethra or positioned adjacent to the skin of a female over the urethra, urine may enter the interior region or chamber of the urine collection device 200 via the opening 210. Accordingly, the urine collection device 200 is configured to receive the fluids into the chamber via the opening 210.

Turning to **FIG. 2B****,** which shows a bottom plan view of the urine collection device 200. The urine collection device 200 can includes a support member 240 secured to the fluid impermeable barrier 205. In the example shown in **FIG. 2B****,** the support member 240 is secured to the outside of the rear or back side of the fluid impermeable barrier 205, distal to the opening 210. In some designs, the support member 240 is secured to the inside of the rear or back side of the fluid impermeable barrier 205. In some designs, the support member 240 is embedded into the rear or back side of the fluid impermeable barrier 205. In some designs, the support member 240 is detachably connected to the back side of the fluid impermeable barrier 205.

The support member 240 is configured to bend or manipulate, and retain the shape manipulated to after manipulation. This support member 240, then, provides a more controllable fluid collection device 200 that can be manipulated into at least a partially compact configuration. For example, the support member 240 may be configured to be manipulated to a generally planar shape and retain the generally planar shape, thereby supporting the back side of the fluid impermeable barrier 205 along an imaginary or theoretical plane. The support member 240 also may be configured to be manipulated to a generally curved or arced shape and retain the generally curved or arced shape, thereby supporting the back side of the fluid impermeable barrier 205 in a generally curved or arced shape (similar to that of the fluid collection device 100 shown in **FIG. 1B****).** The support member 240 also may be configured to be manipulated to a generally rolled compact configuration and retain the generally rolled compact configuration, thereby supporting the back side of the fluid impermeable barrier 205 in a rolled compact configuration (similar to that of the fluid collection device 100 shown in **FIG. 1C****).**

The support member 240 may include a shape memory material such as a shape memory polymer or a metal (*e.g*., shape memory metal). Suitable shape memory materials are composed to adopt an intermediate or permanent shape in response to a stimuli. The stimuli may include an external physical force (*e.g*., bending force), heat, electrical bias, or a magnetic field. While the term "shape memory" is used to describe some of the "shape memory materials" herein, it should be understood that, in some examples, the material modified by the term "shape memory" may not necessarily need to return to a preselected shape upon application of a stimuli, as understood as the classical definition of the "shape memory material." Rather, at least some of the shape memory materials herein may simply hold a selected shape when bent, set, or cured into a specific shape and/or when cooled in a specific shape, regardless of the stimuli applied thereto after. The shape memory materials may be returned to the original shape or changed to a new shape by application of stimuli. For example, a metal wire bent to a first shape may be utilized as the shape memory material, whereinafter the metal wire may be modified to a second shape via physical force applied thereto or via heating.

The shape memory material may include metal, such as an elemental metal, an alloy, or shape memory alloy. Suitable shape memory metals may include standard steels, stainless steel, carbon alloy steel, head treated steel, aluminum, silver, copper, iron, nickel, zinc, tin, beryllium, or the like. Suitable shape memory alloys may include stainless steel; galvanized steel; aluminum alloys; nickel-titanium alloys, such as Nitinol, Ni-Ti-Cu, Ni-Ti, Co, or the like; copper-based alloys such as Cu-Zn-Al, Cu-Al-Ni, Cu-Al-Sn, or the like; Co-Cr-Ni-Mo alloys *(e.g.,* Elgiloy^{®}) or the like; or any other alloy having shape memory characteristics. As explained above, the shape memory metals or alloys may merely be metals or alloys that may be shaped to a selected configuration. In some examples, the shape memory metals or alloys may return to a primary shape when an external stimuli is applied thereto. In some examples, the outer surface of the shape memory metal may be coated with a polymer, anodized, passivated, or otherwise treated to prevent corrosion.

Shape memory polymers ("SMPs") may include polyurethane-based SMPs such as a copolymer (*e.g*., copolyester, polyurethane, polyetherester, etc.) including blocks of one or more of poly(ε-caprolactone), polyethyleneterephthalate (PET), polyethyleneoxide (PEO), polyethylene glycol (PEG), polystyrene, polymethylmethacrylate (PMMA), polybutylmethacrylate (PBMA), poly(N,N-butadiene), poly(N-methyl-N-oxazoline), polytetrahydrofuran, or poly(butylene terephthalate); thermoplastic polymers such as polyether ether ketone (PEEK), nylon, acetal, polytetrafluoroethylene (PTFE), polysulphone, or the like; polynorbonene; other deformable polymers; or any other shape memory polymer.

The support member 240 also may include one or more slots 245 that enhance the flexibility of the support member 240. Although not shown in **FIGS. 1A-1C****,** the urine collection device 100 may include the support member 240 as described above. For example, a support member 240 secured to the fluid collection device 100 may retain to the fluid collection device 100 in the position shown in **FIGS. 1A-1C****.**

Returning to **FIG. 2A****,** the urine collection device also includes a fluid permeable structure 215 positioned within the chamber defined by the fluid impermeable barrier 205 to extend across at least a portion of the opening 210. Unless otherwise noted, the fluid permeable structure 215 may include any of the materials, features, and/or components described above in relation to the fluid permeable structure 115. The fluid permeable structure 215 may be shaped generally complementary to the shape of the chamber of the fluid impermeable barrier 205. For example, when the fluid impermeable barrier 205 includes a generally flat or planar front side in the extended configuration, the fluid permeable structure 215 may include a generally flat or planar surface extending across at least a portion of the opening 210. When the fluid impermeable barrier 205 is generally tubular, as shown in **FIG. 2A****,** the fluid permeable structure 215 may be generally tubular. In some designs, the fluid permeable structure 215 extends to the distal end 225 of the fluid impermeable barrier 205, with the fluid permeable structure 215 tapering with the fluid impermeable barrier 205.

The fluid permeable structure 215 may be configured and sized to roll or fold with the fluid impermeable barrier 205 from the extended configuration to the compact configuration. In some designs, the fluid permeable structure 215 is removable from the chamber of the fluid impermeable barrier 205, and the fluid permeable structure 215 may be absent or separated from the fluid impermeable barrier 205 when the fluid impermeable barrier 205 is in the compact configuration.

The conduit 250 *(e.g.,* a tube) extends through the aperture into the chamber defined by the fluid impermeable barrier 205. The conduit 250 provides fluid communication between the chamber and a fluid storage container (not shown) or a portable vacuum source (not shown). For example, the conduit 250 may directly or indirectly fluidly couple the chamber with the fluid storage container or the portable vacuum source. With the conduit 250 positioned within the fluid permeable structure 215, fluids received in the chamber can be removed through the conduit 250. The conduit 250 may include a continuous single conduit from outside the chamber to inside the chamber. In some designs, the conduit 250 includes the retractable conduit 150 described above.

In use, the opening 210 of the urine collection device 200 may be positioned proximate to a urethra to collect discharged urine or other bodily fluids through the opening 210 and into the fluid permeable structure 215. The support member 240 may bend to retain the fluid collection device 200 in a curve complementary to the individual wearing the urine collection device 200. The discharged urine or other bodily fluids may be wicked through the fluid permeable structure 215 to the conduit 250 for removal from the urine collection device 200 through the conduit 250.

Turning to **FIG. 3****,** which is a side view of a portion of conduit 350. The portion of the conduit 350 shown in **FIG. 3** may be positioned in the chamber of any of the fluid collection devices 100, 200, or 400 (described below). For example, the first portion 160 of the conduit 150 of the urine collection device may include the portion of the conduit 350 shown in **FIG. 3****.** Similarly, the portion of the conduit 250 positioned in the chamber of the urine collection device 200 and/or the portion of the conduit 450 positioned in the chamber of the urine collection device 400 may include the portion of the conduit 350 shown in **FIG. 3****.**

The conduit 350 may include a plurality of eyelets or slots 355. The plurality of eyelets or slots 355 increase the surface area on the conduit 350 for suction of urine or other fluids from the chamber of the urine collection device. With an increase in the surface area on the conduit 350, a reservoir at a distal end of the urine collection device may not be required to collect urine for withdrawal from the urine collection device. Instead, urine may be wicked from the opening of the urine collection device, through the fluid permeable structure to the plurality of eyelets or slots 355 in the conduit 350. Thus, a reservoir in the distal end of the fluid collection device may be absent, allowing for a tapered distal end in some designs, such as the urine collection device 200. A plurality of slots 355 on a portion of the conduit also allow the conduit 350 to be rolled into a compact configuration with the urine collection device. The plurality of eyelets or slots 355 may be positioned intermittently along the entire length of the portion of the conduit 350 within the chamber of the urine collection device.

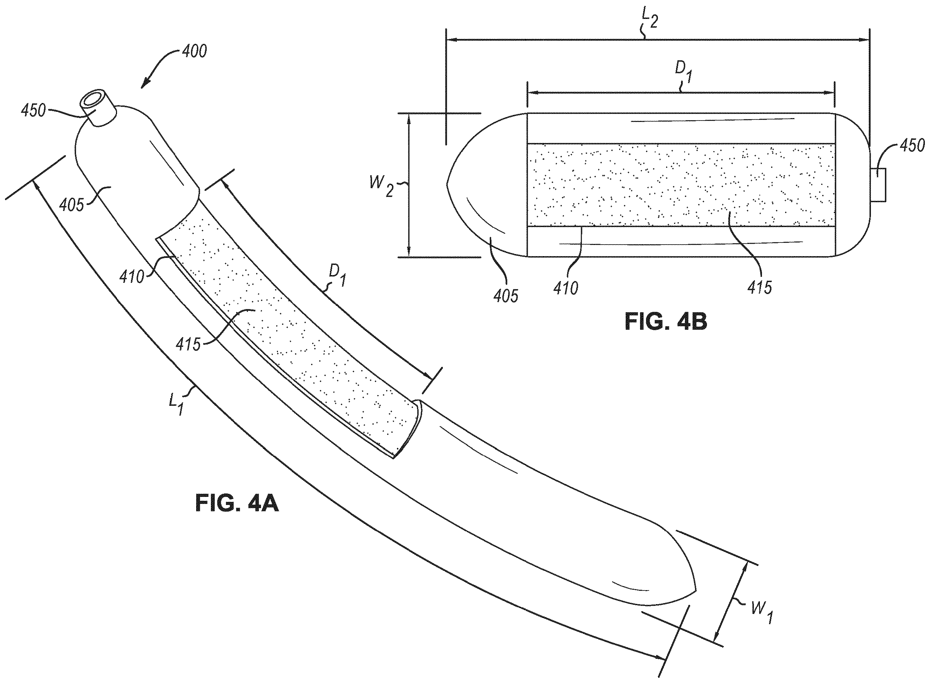

**FIG. 4A** is an isometric view of a urine collection device 400, according to an embodiment of the invention claimed below. The urine collection device 400 is an example of a urine collection device 400 that is configured to receive fluid from a female. Unless otherwise noted, the urine collection device 400 may include any of the materials, components, and/or features described above in relation to the urine collection device 100 or 200. The urine collection device 400 is adjustable between an extended configuration, shown in **FIG. 4A****,** and a compact configuration, shown in **FIG. 4B****.** As shall be described in greater detail below, the axial width and length of the urine collection device 400 may be adjusted by manipulating the urine collection device 400 between the extended configuration and the compact configuration, thereby allowing urine collection device 400 to be adjusted to a size that meets the needs and comforts of an individual user. In particular, a width adjustable urine collection device 400 provides improved ease of securing the urine collection device 400 in place between the legs of the individual using the urine collection device 400.

The urine collection device 400 includes an elongated fluid impermeable barrier 405 that is adjustable between the extended configuration, shown in **FIG. 4A****,** and a compact configuration, shown in **FIG. 4B****.** The fluid impermeable barrier 405 also defines a chamber, an opening 410, and an aperture that is sized and dimensioned to receive a conduit 150 therethrough. The fluid impermeable barrier 405 may temporarily store fluids that have been received through the opening 410 in the chamber. As such, the fluid impermeable barrier 405 substantially prevents the fluids from exiting the portions of the chamber that are spaced from the opening 410. The opening 410 defined by the fluid impermeable barrier 405 is formed in and extends through the fluid impermeable barrier 405, thereby enabling fluids to enter the chamber from outside of the urine collection device 400. The opening 410 can be configured to be positioned adjacent to a female urethra or positioned adjacent to the skin of a female over the urethra. With the urine collection device 400 positioned proximate to the female urethra or positioned adjacent to the skin of a female over the urethra, urine may enter the interior region or chamber of the urine collection device 400 via the opening 410. Accordingly, the urine collection device 400 is configured to receive the fluids into the chamber via the opening 410.

The fluid impermeable barrier 405 is flexible, allowing the urine collection device 400 to bend or curve when positioned against the body of a wearer. The fluid impermeable barrier 405 can be formed of any suitable fluid impermeable materials described above in relation to the fluid impermeable barriers 105 or 205, such as a fluid impermeable polymer (e.g., silicone, polypropylene, polyethylene, polyethylene terephthalate, a polycarbonate, etc.), polyurethane films, thermoplastic elastomer, oil, another suitable material, or combinations thereof.

The fluid impermeable barrier 405 may be generally cylindrical or tubular in shape. In the generally cylindrical shape, the opening 410 may be defined by two arched borders and two longitudinal borders extending between the two arched borders. In other embodiments, the fluid impermeable barrier 405 may include a substantially flat or planar area, such as a substantially flat back side and front side defining the opening 410 having a substantially planar profile when the fluid impermeable barrier 405 is in the extended configuration shown in **FIG. 4A****.**

According to the invention, the fluid impermeable barrier 405 also is adjustable between an extended configuration, shown in **FIG. 4A****,** and a compact configuration, shown in **FIG. 4B****.** In the extended configuration, the fluid impermeable barrier 405 includes a first axial length L₁ and a first width W₁, with the opening 410 having a longitudinal dimension D₁. When the fluid impermeable barrier 405 is manipulated to the compact configuration shown in **FIG. 4B****,** the fluid impermeable barrier 405 includes a second axial length L₂ that is less than the first axial length L₁, a second width W₂ that is greater than the first width W₁. In the compact configuration, the longitudinal dimension D₁ of the opening 410 is substantially equal to the longitudinal dimension D₁ of the opening 410 in the extended configuration.

The fluid impermeable barrier 405 may include one or more support members secured to the fluid impermeable barrier 405. The one or more support members may be secured to an inner surface of the fluid impermeable barrier 405 and/or at least partially embedded in the fluid impermeable barrier 405. For example, the one or more support members may include bendable plastic, metal, other materials, or some combination thereof at least partially embedded in the fluid impermeable barrier 405.

The one or more support members are positioned on the fluid impermeable barrier 405 to increase the width of the fluid impermeable barrier 405 from the first width W₁ to the second width W₂ when the axial length of the fluid impermeable barrier 405 is compressed from the first axial length L₁ to the second axial length L₂ of the compact configuration. The one or more support members are further positioned on the fluid impermeable barrier 405 to decrease the width of the fluid impermeable barrier 405 from the second width W₂ to the first width W₁ when the axial length of the fluid impermeable barrier 405 is extended from the second axial length L₂ to the first axial length L₁ of the extended configuration. According to the invention, the support member is further positioned on the fluid impermeable barrier 405 and configured to allow a user to stop at any desired width between W₁ and W₂ to meet the needs and physiological dimensions of the individual wearing the urine collection device 400.

The one or more support members can include at least one of a hinge system skeleton, one or more adjustable railings, one or more telescoping tubes, or a gear system configured to increase the width of the fluid impermeable barrier 405 as the axial length of the fluid impermeable barrier 405 is shortened, and also decrease the width of the fluid impermeable barrier 405 as the axial length of the fluid impermeable barrier 405 is extended. In some examples, the one or more support members may include a framework of hinged rods that can extend outward to increase the width of the fluid impermeable barrier 405. Accordingly, the one or more support members to exert a pressure on the fluid impermeable barrier 405 to expand or stretch the fluid impermeable barrier 405 laterally when the fluid impermeable barrier 405 is compressed longitudinally along the axial length. In some embodiments, a distal portion of the fluid impermeable barrier may include one or more telescoping portion configured to extend or collapse the fluid impermeable barrier between L₁ and L₂.

The urine collection device also includes fluid permeable structure 415 positioned within the chamber defined by the fluid impermeable barrier 405 to extend across at least a portion of the opening 410. The fluid permeable structure 415 may be shaped generally complementary to the shape of the chamber of the fluid impermeable barrier 405. For example, when the fluid impermeable barrier 405 is generally tubular, the fluid permeable structure 415 may be generally tubular. When the fluid impermeable barrier 405 includes a generally flat or planar front side in the extended configuration, the fluid permeable structure 415 may include a generally flat or planar surface extending across at least a portion of the opening 410.

The fluid permeable structure 415 can be configured to wick any fluid away from the opening 410, thereby preventing the fluid from escaping the chamber of the fluid impermeable barrier 405. The fluid permeable structure 415 also can wick the fluid generally towards an interior of the chamber. A portion of the fluid permeable structure 415 can define a portion of an outer surface of the urine collection device 400. Specifically, the portion of the fluid permeable structure 415 defining the portion of the outer surface of the urine collection device 400 can be the portion of the fluid permeable structure 415 exposed by the opening 410 defined by the fluid impermeable barrier 405 that contacts the user. The fluid permeable structure 415 can include any materials, components, or features described above in relation to the fluid permeable structures 115 and 215.

In some embodiments, the fluid permeable structure 415 is secured to the fluid impermeable barrier 405 proximate to the opening 410. With the fluid permeable structure 415 secured to the fluid impermeable barrier 405, undesired gaps are not formed between the fluid permeable structure 415 and the fluid impermeable barrier 405 at the opening 410 when the width of the fluid impermeable barrier 405 is increased. In some embodiments, the width of the fluid permeable structure 415 may increase or expand corresponding to the increase of the width of the fluid impermeable barrier 405, and may decrease or compress corresponding to the decrease of the width of the fluid impermeable barrier 405. The fluid impermeable barrier 405 also may include foam or other compressible material proximate the opening to prevent undesired gaps between the fluid permeable structure 415 and the fluid impermeable barrier 405 at the opening 410 when the width of the fluid impermeable barrier 405 is increased.

A reservoir inside the chamber may be defined at least partially by the fluid impermeable barrier 405 and the fluid permeable structure 415. The reservoir is void of material and positioned distal to the aperture. In some embodiments, a volume of the reservoir is fixed, and does not change when the fluid impermeable barrier is manipulated between the extended configuration and the compact configuration. For example, the fluid impermeable barrier 405 may include a reservoir wall positioned within the chamber at a fixed distance from the fluid permeable structure 415. The reservoir, then, may be defined at least partially by the fluid permeable structure 415 and the reservoir wall of the fluid impermeable barrier 405. In some embodiments, a fluid impermeable cap is secured to an end of the fluid permeable structure 415 distal to the aperture, with a space or gap between the fluid permeable structure 415 and a reservoir wall of the fluid impermeable cap. The reservoir, then, may be defined at least partially by the end of the fluid permeable structure 415 and the reservoir wall of the fluid impermeable cap. In some embodiments, fluid impermeable barrier may include a movable reservoir wall that moves with as the axial length of the fluid impermeable barrier 405 increases or decreases. In these and other embodiments, then, the volume of the reservoir may change as the axial length of the fluid impermeable barrier 405 increases or decreases.

The conduit 450 extends through the aperture into the chamber defined by the fluid impermeable barrier 405. In some embodiments, the conduit 450 extends to the reservoir. For example, the conduit 450 may extend at least partially into the reservoir, may be generally flush with the end of the fluid permeable structure 415, or recessed from the fluid reservoir. The conduit 450 provides fluid communication between the chamber and/or the reservoir and a fluid storage container (not shown) or a portable vacuum source (not shown). For example, the conduit 450 may directly or indirectly fluidly couple the chamber and/or the reservoir with the fluid storage container or the portable vacuum source. With the conduit 450 positioned within the fluid permeable structure 415, fluids received in the chamber and/or the reservoir can be removed through the conduit 450. The conduit 450 may include any of the conduits described herein, including the retractable conduit 150 and/or the conduit 350 having a plurality of eyelets or slots 355.

In use, the urine collection device 400 may be positioned between the legs of the individual wearing the urine collection device 400. The width of the fluid impermeable barrier 405 may be increased or decreased to more comfortably between the legs of the individual wearing the urine collection device 400 by compressing or extending, respectively, an axial length of the urine collection device 400. The opening 410 of the urine collection device 400 may be positioned proximate to a female urethra to collect discharged urine or other bodily fluids through the opening 410 and into the fluid permeable structure 415. The discharged urine or other bodily fluids may be wicked through the fluid permeable structure 415 to the conduit 450 for removal from the urine collection device 400 through the conduit 450.

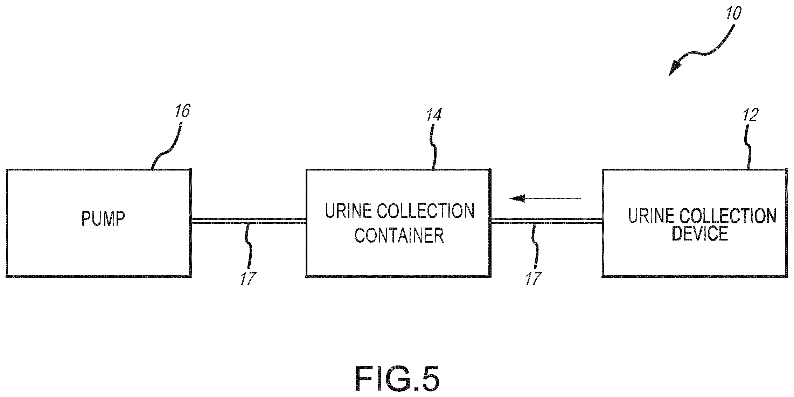

**FIG. 5** is a block diagram of a fluid collection system 10, according to an embodiment. The fluid collection system 10 may be included in embodiments of fluid collection systems described herein. The system 10 includes a fluid *(e.g.,* urine) collection device 12 *(e.g.,* any of the urine collection devices disclosed herein, including urine collection device 100, 200, 400), a urine collection container 14, and a pump 16 (or vacuum source). The fluid collection device 10, the urine collection container 14, and the pump 16 may be fluidly coupled to each other via one or more conduits 17. For example, fluid collection device 10 may be operably coupled to one or more of the urine collection container 14 or the pump 16 via the conduit 17. In some embodiments, the pump 16 may be secured directly to the urine collection container 14. Fluid (e.g., urine or other bodily fluids) collected in the fluid collection device 10 may be removed from the fluid collection device 10 via the conduit 17 secured to the fluid collection device 12. Suction force may be introduced into the chamber of the fluid collection device 12 via the inlet of the conduit 17 responsive to suction (e.g., vacuum) force applied at the outlet of the conduit 17.

The suction force may be applied to the outlet of the conduit 17 by the pump 16 either directly or indirectly. The suction force may be applied indirectly via the urine collection container 14. For example, the outlet of the conduit 17 may be disposed within or fluidly coupled to an interior region of the urine collection container 14 and an additional conduit 17 may extend from the urine collection container 14 to the pump 16. Accordingly, the pump 16 may apply suction to the fluid collection device 12 via the urine collection container 14. The suction force may be applied directly via the pump 16. For example, the outlet of the conduit 17 may be disposed within the pump 16. An additional conduit 17 may extend from the pump 16 to a point outside of the fluid collection device 12, such as to the urine collection container 14. In such examples, the pump 16 may be disposed between the fluid collection device 12 and the urine collection container 14.

The urine collection container 14 is sized and shaped to retain a fluid therein. The urine collection container 14 may include a bag (e.g., drainage bag), a bottle or cup *(e.g.,* collection jar), or any other enclosed container for storing bodily fluid(s) such as urine. In some examples, the conduit 17 may extend from the fluid collection device 12 and attach to the urine collection container 14 at a first point therein. An additional conduit 17 may attach to the urine collection container 14 at a second point thereon and may extend and attach to the pump 16. Accordingly, a vacuum (e.g., suction) may be drawn through fluid collection device 12 via the urine collection container 14. Fluid, such as urine, may be drained from the fluid collection device 12 using the pump 16.

The pump 16 or vacuum source may include one or more of a manual vacuum pump, and electric vacuum pump, a diaphragm pump, a centrifugal pump, a displacement pump, a magnetically driven pump, a peristaltic pump, or any pump configured to produce a vacuum. The pump 16 may provide a vacuum or suction to remove fluid from the fluid collection device 12. In some examples, the pump 16 may be powered by one or more of a power cord *(e.g.,* connected to a power socket), one or more batteries, or even manual power *(e.g.,* a hand operated vacuum pump). In some examples, the pump 16 may be sized and shaped to fit outside of, on, or within the fluid collection device 12. For example, the pump 16 may include one or more miniaturized pumps or one or more micro pumps. The vacuum sources disclosed herein may include one or more of a switch, a button, a plug, a remote, or any other device suitable to activate the pump 16.

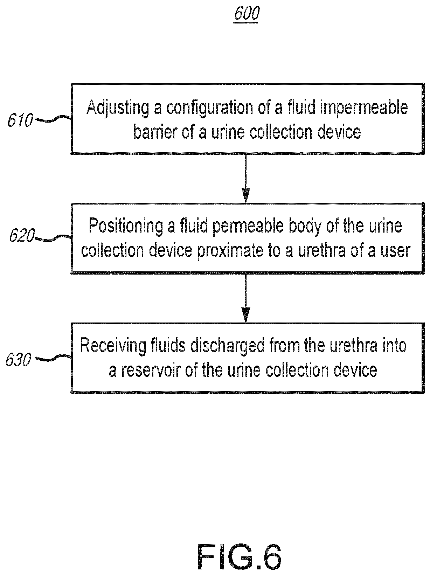

**FIG. 6** is a flow diagram of a method 600 for collecting urine. The method 600 includes an act 610 of adjusting a configuration of a fluid impermeable barrier of a urine collection device to fit between legs of a user. The fluid impermeable barrier at least partially defines a chamber, an aperture configured to receive a conduit therethrough, and an opening. The method includes an act 620 of positioning a fluid permeable structure of the urine collection device at least proximate to a urethra of a user, the fluid permeable structure extending at least partially across the opening defined by the fluid impermeable barrier. The method 600 includes an act 630 of receiving fluids discharged from the urethra into the chamber. The method 600 may also include an act of promoting flow of urine from the chamber through a conduit fluidly coupled to the reservoir.

The act 610 of adjusting a configuration of a fluid impermeable barrier may include unrolling and/or unfolding the fluid impermeable barrier and the fluid permeable structure from a compact configuration to an extended configuration. For example, the urine collection device of the method 600 may include the urine collection device 100 or 200. The method 600 may include an act of retaining the fluid impermeable barrier in the compact configuration or the extended configuration with a support member secured to the fluid impermeable barrier.

The act 610 of adjusting a configuration of a fluid impermeable barrier includes manipulating a support member secured to the fluid impermeable barrier to adjust at least one of an axial length of the fluid impermeable barrier or a width of the fluid impermeable barrier. For example, the urine collection device of the method 600 may include the urine collection device 400. In accordance with the present invention, manipulating a support member secured to the fluid impermeable barrier adjusts an axial length of the fluid impermeable barrier and a width of the fluid impermeable barrier includes manipulating the support member to decrease the axial length of the fluid impermeable barrier and increase the width of the fluid impermeable barrier. In some embodiments, the method 600 also includes an act of expanding the fluid permeable structure to extend across the opening when the width of the fluid impermeable barrier is increased.

In some embodiments, the method 600 also includes an act of extending a length of a conduit positioned at least partially within the chamber by pulling a portion of the conduit slidably connected to a second portion of the conduit. For example, the urine collection device of the method 600 may include the conduit 150.

The acts of the method 600 described above are for illustrative purposes. For example, the acts of the method 600 can be performed in different orders, split into multiple acts, modified, supplemented, or combined. One or more of the acts of the method 600 can be omitted from the method 600. Any of the acts of the method 600 can include using any of the urine collection systems disclosed herein.

As used herein, the term "about" or "substantially" refers to an allowable variance of the term modified by "about" or "substantially" by ±10% or ±5%. Further, the terms "less than," "or less," "greater than," "more than," or "or more" include, as an endpoint, the value that is modified by the terms "less than," "or less," "greater than," "more than," or "or more."

## Claims

1. A cylindrical urine collection device (400) to be worn by a female, the device comprising:
a fluid impermeable barrier (405) at least partially defining a chamber, an aperture configured to receive a conduit therethrough, and an opening through (410) which urine is received into the chamber, the opening having a longitudinal dimension (D₁) lengthwise of the device and a width transverse to its length; and
a fluid permeable structure (415) positioned within the chamber to extend across at least a portion of the opening, the fluid permeable structure being configured to wick fluid away from the opening; and
further comprising one or more support members secured to the fluid impermeable barrier, the support member configured to retain the fluid impermeable barrier in an extended configuration and in a compact configuration so that the fluid impermeable barrier is adjustable between the extended configuration and the compact configuration; and wherein:
the fluid impermeable barrier includes:
a first axial length (L₁) in the extended configuration;
a second axial length (L₂) in the compact configuration, the first axial length being greater than the second axial length;
a first width (W₁) in the extended configuration; and
a second width (W₂) in the compact configuration, the second width being greater than the first width;
the support member expands the fluid impermeable barrier from the first width to the second width responsive to manipulation of the fluid impermeable barrier from the extended configuration to the compact configuration, with the longitudinal dimension (D₁) of the opening in the compact configuration and the extended configuration being substantially equal; and wherein:
the support member allows a user to stop at any desired width between W₁ and W₂ to meet the needs and physiological dimensions of the individual wearing the urine collection device (400).

2. The urine collection device of claim 1, wherein the fluid permeable structure includes an outer gauze portion and an inner porous nylon portion configured to wick fluid from the opening to the chamber of the fluid impermeable barrier.

3. The urine collection device of claim 1 or 2, wherein the support member includes at least one of a hinge system skeleton, one or more adjustable metal railings, or a gear system.

4. The urine collection device of claim 1 or 2, wherein the one or more support members includes one or more telescoping tubes.

5. The urine collection device of any of the preceding claims, wherein the fluid permeable structure is secured to the fluid impermeable barrier proximate to the opening.

6. The urine collection device of any of the preceding claims, wherein the fluid permeable structure (415) expands to extend across the opening (410) when the fluid impermeable barrier (405) is manipulated from the extended configuration to the compact configuration.

7. The urine collection device of any of the preceding claims, further comprising a conduit (450) extending through the aperture at least partially into the chamber, and wherein at least a portion of conduit positioned in the chamber includes a plurality of slots or eyelets (355) sized to pull urine from the chamber into the conduit when the conduit is fluidly coupled to a vacuum.

8. The urine collection device of any of the preceding claims, wherein the conduit includes a first portion positioned at least partially within the chamber and a second portion slidably connected to the first portion, the second portion at least partially positioned outside the chamber and at least partially retractable within the chamber.

9. The urine collection device of any one of the preceding claims, wherein the fluid permeable structure includes a wicking layer and a transporting layer configured to wick fluid from the opening to the chamber of the fluid impermeable barrier.

10. The urine collection device as claimed in any one of the preceding claims, wherein the fluid impermeable barrier (405) is tubular and the fluid permeable structure (415) is tubular and complementary to the shape of the chamber.

11. The urine collection device as claimed in any one of the preceding claims, wherein the fluid impermeable barrier (405) includes a flat or planar front side in the extended configuration and the fluid permeable structure (415) includes a flat or planar surface extending across at least a portion of the opening (410).

12. The urine collection device as claimed in any one of the preceding claims, wherein the fluid impermeable barrier (405) includes compressible material proximate the opening to prevent undesired gaps between the fluid permeable structure (415) and the fluid impermeable barrier (405) at the opening (410) when the width of the fluid impermeable barrier (405) is increased.

## Patentansprüche

1. Zylindrische Urinsammelvorrichtung (400), die von einer Frau zu tragen ist, wobei die Vorrichtung Folgendes umfasst:
eine flüssigkeitsundurchlässige Barriere (405), die zumindest teilweise eine Kammer definiert, eine Öffnung, die so konfiguriert ist, dass sie eine Leitung durch sie hindurch aufnimmt, und eine Öffnung (410), durch die Urin in die Kammer aufgenommen wird, wobei die Öffnung eine Längsabmessung (D₁) in Längsrichtung der Vorrichtung und eine Breite quer zu ihrer Länge aufweist; und
eine flüssigkeitsdurchlässige Struktur (415), die in der Kammer positioniert ist, um sich über mindestens einen Abschnitt der Öffnung zu erstrecken, wobei die flüssigkeitsdurchlässige Struktur so konfiguriert ist, dass sie Flüssigkeit von der Öffnung wegtransportiert; und
weiter umfassend ein oder mehrere Stützelemente, die an der flüssigkeitsundurchlässigen Barriere befestigt sind, wobei das Stützelement so konfiguriert ist, dass es die flüssigkeitsundurchlässige Barriere in einer ausgefahrenen Konfiguration und in einer kompakten Konfiguration hält, sodass die flüssigkeitsundurchlässige Barriere zwischen der ausgefahrenen Konfiguration und der kompakten Konfiguration einstellbar ist; und wobei:
die flüssigkeitsundurchlässige Barriere Folgendes einschließt:
eine erste axiale Länge (L₁) in der ausgefahrenen Konfiguration;
eine zweite axiale Länge (L₂) in der kompakten Konfiguration, wobei die erste axiale Länge größer ist als die zweite axiale Länge;
eine erste Breite (W₁) in der ausgefahrenen Konfiguration; und
eine zweite Breite (W₂) in der kompakten Konfiguration, wobei die zweite Breite größer ist als die erste Breite;
das Stützelement die flüssigkeitsundurchlässige Barriere als Reaktion auf die Manipulation der flüssigkeitsundurchlässigen Barriere von der ausgefahrenen Konfiguration in die kompakte Konfiguration von der ersten Breite auf die zweite Breite ausdehnt, wobei die Längsabmessung (D₁) der Öffnung in der kompakten Konfiguration und in der ausgefahrenen Konfiguration im Wesentlichen gleich ist; und wobei:
das Stützelement es einem Benutzer ermöglicht, in jeder gewünschten Breite zwischen W₁ und W₂ anzuhalten, um den Bedürfnissen und physiologischen Abmessungen der Person, die die Urinsammelvorrichtung (400) trägt, zu entsprechen.

2. Urinsammelvorrichtung nach Anspruch 1, wobei die flüssigkeitsdurchlässige Struktur einen äußeren Mullabschnitt und einen inneren porösen Nylonabschnitt einschließt, der so konfiguriert ist, dass er Flüssigkeit von der Öffnung in die Kammer der flüssigkeitsundurchlässigen Barriere transportiert.

3. Urinsammelvorrichtung nach Anspruch 1 oder 2, wobei das Stützelement mindestens eines von einem Scharniersystem-Skelett, einem oder mehreren einstellbaren Metallgeländern oder einem Getriebesystem einschließt.

4. Urinsammelvorrichtung nach Anspruch 1 oder 2, wobei das eine oder die mehreren Stützelemente ein oder mehrere Teleskopröhren einschließen.

5. Urinsammelvorrichtung nach einem der vorstehenden Ansprüche, wobei die flüssigkeitsdurchlässige Struktur an der flüssigkeitsundurchlässigen Barriere in der Nähe der Öffnung befestigt ist.

6. Urinsammelvorrichtung nach einem der vorstehenden Ansprüche, wobei die flüssigkeitsdurchlässige Struktur (415) sich ausdehnt, um sich über die Öffnung (410) zu erstrecken, wenn die flüssigkeitsundurchlässige Barriere (405) von der ausgefahrenen Konfiguration in die kompakte Konfiguration manipuliert wird.

7. Urinsammelvorrichtung nach einem der vorstehenden Ansprüche, weiter umfassend eine Leitung (450), die sich durch die Öffnung zumindest teilweise in die Kammer erstreckt, und wobei mindestens ein Abschnitt der in der Kammer positionierten Leitung eine Vielzahl von Schlitzen oder Ösen (355) einschließt, die so bemessen sind, dass sie Urin aus der Kammer in die Leitung ziehen, wenn die Leitung mit einem Vakuum in Fluidverbindung steht.

8. Urinsammelvorrichtung nach einem der vorstehenden Ansprüche, wobei die Leitung einen ersten Abschnitt, der zumindest teilweise innerhalb der Kammer positioniert ist, und einen zweiten Abschnitt einschließt, der gleitend mit dem ersten Abschnitt verbunden ist, wobei der zweite Abschnitt zumindest teilweise außerhalb der Kammer positioniert ist und zumindest teilweise in die Kammer einziehbar ist.

9. Urinsammelvorrichtung nach einem der vorstehenden Ansprüche, wobei die flüssigkeitsdurchlässige Struktur eine Saugschicht und eine Transportschicht einschließt, die so konfiguriert sind, dass sie Flüssigkeit von der Öffnung zur Kammer der flüssigkeitsundurchlässigen Barriere leiten.

10. Urinsammelvorrichtung nach einem der vorstehenden Ansprüche, wobei die flüssigkeitsundurchlässige Barriere (405) röhrenförmig ist und die flüssigkeitsdurchlässige Struktur (415) röhrenförmig und komplementär zur Form der Kammer ist.

11. Urinsammelvorrichtung nach einem der vorstehenden Ansprüche, wobei die flüssigkeitsundurchlässige Barriere (405) eine flache oder ebene Vorderseite in der ausgefahrenen Konfiguration einschließt und die flüssigkeitsdurchlässige Struktur (415) eine flache oder ebene Oberfläche einschließt, die sich über mindestens einen Abschnitt der Öffnung (410) erstreckt.

12. Urinsammelvorrichtung nach einem der vorstehenden Ansprüche, wobei die flüssigkeitsundurchlässige Barriere (405) komprimierbares Material in der Nähe der Öffnung einschließt, um unerwünschte Lücken zwischen der flüssigkeitsdurchlässigen Struktur (415) und der flüssigkeitsundurchlässigen Barriere (405) an der Öffnung (410) zu verhindern, wenn die Breite der flüssigkeitsundurchlässigen Barriere (405) erhöht wird.

## Revendications

1. Dispositif de collecte d'urine (400) cylindrique destiné à être porté par une femme, le dispositif comprenant :
une barrière imperméable aux fluides (405) définissant au moins partiellement une chambre, un orifice configuré pour recevoir un conduit à travers celle-ci, et une ouverture (410) à travers laquelle l'urine est reçue dans la chambre, l'ouverture présentant une dimension longitudinale (D₁) dans le sens de la longueur du dispositif et une largeur transversale à sa longueur ; et
une structure perméable aux fluides (415) positionnée à l'intérieur de la chambre pour s'étendre à travers au moins une partie de l'ouverture, la structure perméable aux fluides étant configurée pour évacuer le fluide par l'ouverture ; et
comprenant en outre un ou plusieurs éléments de support fixés à la barrière imperméable aux fluides, l'élément de support étant configuré pour maintenir la barrière imperméable aux fluides dans une configuration étendue et dans une configuration compacte de sorte que la barrière imperméable aux fluides soit réglable entre la configuration étendue et la configuration compacte ; et dans lequel :
la barrière imperméable aux fluides inclut :
une première longueur axiale (L₁) dans la configuration étendue ;
une seconde longueur axiale (L₂) dans la configuration compacte, la première longueur axiale étant supérieure à la seconde longueur axiale ;
une première largeur (W₁) dans la configuration étendue ; et
une seconde largeur (W₂) dans la configuration compacte, la seconde largeur étant supérieure à la première largeur ;
l'élément de support dilate la barrière imperméable aux fluides de la première largeur à la seconde largeur en réponse à une manipulation de la barrière imperméable aux fluides de la configuration étendue à la configuration compacte, avec la dimension longitudinale (D₁) de l'ouverture dans la configuration compacte et la configuration étendue étant substantiellement égale ; et dans lequel :
l'élément de support permet à un utilisateur de s'arrêter à toute largeur souhaitée entre W₁ et W₂ pour répondre aux besoins et dimensions physiologiques de l'individu portant le dispositif de collecte d'urine (400).

2. Dispositif de collecte d'urine selon la revendication 1, dans lequel la structure perméable aux fluides inclut une partie gaze externe et une partie nylon poreux interne configurées pour évacuer le fluide par l'ouverture vers la chambre de la barrière imperméable aux fluides.

3. Dispositif de collecte d'urine selon la revendication 1 ou 2, dans lequel l'élément de support inclut au moins un parmi un squelette de système d'articulation, une ou plusieurs rampes métalliques réglables, ou un système d'engrenages.

4. Dispositif de collecte d'urine selon la revendication 1 ou 2, dans lequel les un ou plusieurs éléments de support incluent un ou plusieurs tubes télescopiques.

5. Dispositif de collecte d'urine selon l'une quelconque des revendications précédentes, dans lequel la structure perméable aux fluides est fixée à la barrière imperméable aux fluides à proximité de l'ouverture.

6. Dispositif de collecte d'urine selon l'une quelconque des revendications précédentes, dans lequel la structure perméable aux fluides (415) se dilate pour s'étendre à travers l'ouverture (410) lorsque la barrière imperméable aux fluides (405) est manipulée de la configuration étendue à la configuration compacte.

7. Dispositif de collecte d'urine selon l'une quelconque des revendications précédentes, comprenant en outre un conduit (450) s'étendant à travers l'orifice au moins partiellement dans la chambre, et dans lequel au moins une partie de conduit positionnée dans la chambre inclut une pluralité de fentes ou d'oeillets (355) dimensionnés pour attirer l'urine de la chambre jusque dans le conduit lorsque le conduit est couplé fluidiquement à un vide.

8. Dispositif de collecte d'urine selon l'une quelconque des revendications précédentes, dans lequel le conduit inclut une première partie positionnée au moins partiellement à l'intérieur de la chambre et une seconde partie raccordée de manière coulissante à la première partie, la seconde partie étant au moins partiellement positionnée à l'extérieur de la chambre et au moins partiellement rétractable à l'intérieur de la chambre.

9. Dispositif de collecte d'urine selon l'une quelconque des revendications précédentes, dans lequel la structure perméable aux fluides inclut une couche d'évacuation et une couche de transport configurées pour évacuer le fluide par l'ouverture vers la chambre de la barrière imperméable aux fluides.

10. Dispositif de collecte d'urine selon l'une quelconque des revendications précédentes, dans lequel la barrière imperméable aux fluides (405) est tubulaire et la structure perméable aux fluides (415) est tubulaire et complémentaire à la forme de la chambre.

11. Dispositif de collecte d'urine selon l'une quelconque des revendications précédentes, dans lequel la barrière imperméable aux fluides (405) inclut un côté avant plat ou planaire dans la configuration étendue et la structure perméable aux fluides (415) inclut une surface plate ou planaire s'étendant à travers au moins une partie de l'ouverture (410).

12. Dispositif de collecte d'urine selon l'une quelconque des revendications précédentes, dans lequel la barrière imperméable aux fluides (405) inclut un matériau compressible à proximité de l'ouverture pour empêcher des interstices non souhaités entre la structure perméable aux fluides (415) et la barrière imperméable aux fluides (405) au niveau de l'ouverture (410) lorsque la largeur de la barrière imperméable aux fluides (405) est augmentée.
